Europäisches Patentamt

European Patent Office

Office européen des brevets

(11) Publication number: **0 313 215**
**A2**

## (12) EUROPEAN PATENT APPLICATION

(21) Application number: **88308723.1**

(51) Int. Cl.⁴: **A61L 9/12**

(22) Date of filing: **20.09.88**

The title of the invention has been amended (Guidelines for Examination in the EPO, A-III, 7.3).

(30) Priority: **21.09.87 GB 8722188**

(43) Date of publication of application:
**26.04.89 Bulletin 89/17**

(84) Designated Contracting States:
**AT BE CH DE ES FR GB IT LI NL**

(71) Applicant: **THE WELLCOME FOUNDATION LIMITED**
**183-193 Euston Road**
**London NW1 2BP(GB)**

(72) Inventor: **Tipler, Brian John**
**19 Bendemeer Road**
**Putney London SW15 1JX(GB)**

(74) Representative: **Garrett, Michael et al**
**The Wellcome Research Laboratories Group**
**Patents and Agreements Langley Court**
**Beckenham Kent BR3 3BS(GB)**

(54) **Dispensing apparatus.**

(57) Controlled release of fluent material to be dispensed (e.g. in an air freshener) is achieved by encapsulating the material in pockets in a web (12) and rupturing the pockets (e.g. with a brush 30) in a dispensing station (12c), the released material being entrained by air moved past the dispensing station by a fan (10). Advance of the web can be controlled by a motor (24). A stand-by web (14) can be provided for use when the current web (12) is exhausted.

Fig.1.

## IMPROVED DISPENSING METHOD AND APPARATUS

This invention relates to an improved method of dispensing fluent material, a dispenser for carrying out the method and web material for use in the dispenser.

It is known to provide units for adding odorising or deodorising agents to the ambient air in washrooms, toilets and the like and this invention is expected to have particular, but not exclusive, utility in that application.

According to one aspect of the present invention, a method of dispensing a fluent material, particularly a volatile liquid required for treatment of ambient air, comprises conveying a web incorporating a multitude of frangible material-filled pockets past a dispensing station and releasing the fluent material from the pockets by rupturing them at the dispensing station.

In a simple embodiment, the web includes a substantially uniform volume of fluent material per unit of web length and means is provided to advance the web through the dispensing station at a uniform rate.

The, or part of the, web may be of absorbent material so that on rupture of each pocket, the released material flows onto and is absorbed by the web or web part (e.g. for subsequent release into the ambient air). Means can be provided to move air past or through the web to entrain vapour from the released material.

The advance of the web can be controlled on the basis of the effluxion of time or on the happening of a given event. Thus for example where odorising/deodorising of the air in a given room is required, a dispensing unit can be located in that room and the web can be advanced through the dispensing station at a rate which produces a required rate of release of vapour-generating material. Where a uniform rate of release is required throughout the 24 hours of each day, a web with a uniform distribution of liquid-filled pockets can be advanced at a uniform rate through the dispensing station. Where different rates are required at different times in a day or during different days in a week either the rate at which pockets are ruptured is made to be different by moving a web with a constant volume of pocketed material per unit length of web at different rates, or a web with a gradation of volumes of pocketed material per unit length is moved at a uniform rate through the dispensing station. Thus a web could be provided with different concentrations per unit of web area of equal sized pockets over regions indicated as being suitable for day/night use and/or weekday/weekend use and a service person ensures the correct part of the web passes through the dispensing station at the correct time.

Another regime is to advance the web a suitable amount on each happening of a specified event (e.g. the opening of a door, turning on of a light, interrupting of a light beam or flushing of a toilet). In these and other ways, the method of the invention can be used to match the release of vapour-generating material to the actual or expected use of a facility or room to properly condition the air therein.

Following dispensing, the released material can be used in a variety of ways. The presently-designed units release a liquid for incorporation into the ambient atmosphere of a room but it will be appreciated the invention can also be used, for example, to add material to a fluid (e.g. a liquid) flowing in a pipe or duct.

According to a further aspect of the invention there is provided a dispensing unit for dispensing a volatile material into a flow of air, comprising means to advance a web incorporating a multitude of frangible material-filled pockets through a dispensing station, means to rupture the pockets in the dispensing station and means to generate a flow of air part the material released from the ruptured pockets.

A dispenser in accordance with the invention could include two webs, a second web acting as a stand-by for use when the first web has been passed through the dispensing station. A leading end of the stand-by web could be adhered to the trailing end of the first web so that both pass in sequence through the same dispensing station but two dispensing stations could be provided (e.g. side-by-side) one for each web. The dispenser could include a prime mover (e.g. an electric or clockwork motor) for advancing the web(s).

The pockets can be of any convenient size and density of positioning on the web. Microcapsules are a suitable form of pocket since there is a well established technology to produce sheets of microencapsulated fluent material.

All pockets can contain the same fluent material but where more than one substance needs to be dispensed it is not ruled out that pockets housing different fluent materials can be arranged on the one web. The web can be of a fibrous (woven or non-woven) material such as paper and/or of a unitary sheet material (e.g. a foil or a plastics sheet). It is desirably rolled up on a core and rerolled after having passed through the dispensing station.

The dispensing station can include a pair of nip rollers, a nip roller and a confronting pressure plate, a pair of pressure plates or a rotating brush or the like pocket-rupturing member. A fan (continuously or intermittently operable) can be provided to encourage distribution of vapour from the material leaving the ruptured pockets.

A web containing pockets of fluent material for use in the method or dispensing unit of this invention represents a further feature of the invention.

Embodiments of dispensing unit in accordance with this invention will now be described, by way of example, with reference to the accompanying drawings, in which:

Figure 1 is a partially exploded, schematic perspective view of the component parts of a first embodiment of dispensing unit,

Figure 2 is a diagram of the basic principle behind a second embodiment of dispensing unit in accordance with this invention, and

Figure 3 is an enlarged view of part of the unit of Figure 1.

The dispensing unit will include a casing (not shown) for wall mounting, which casing includes louvres through which air is drawn into and expelled from the casing by a fan 10.

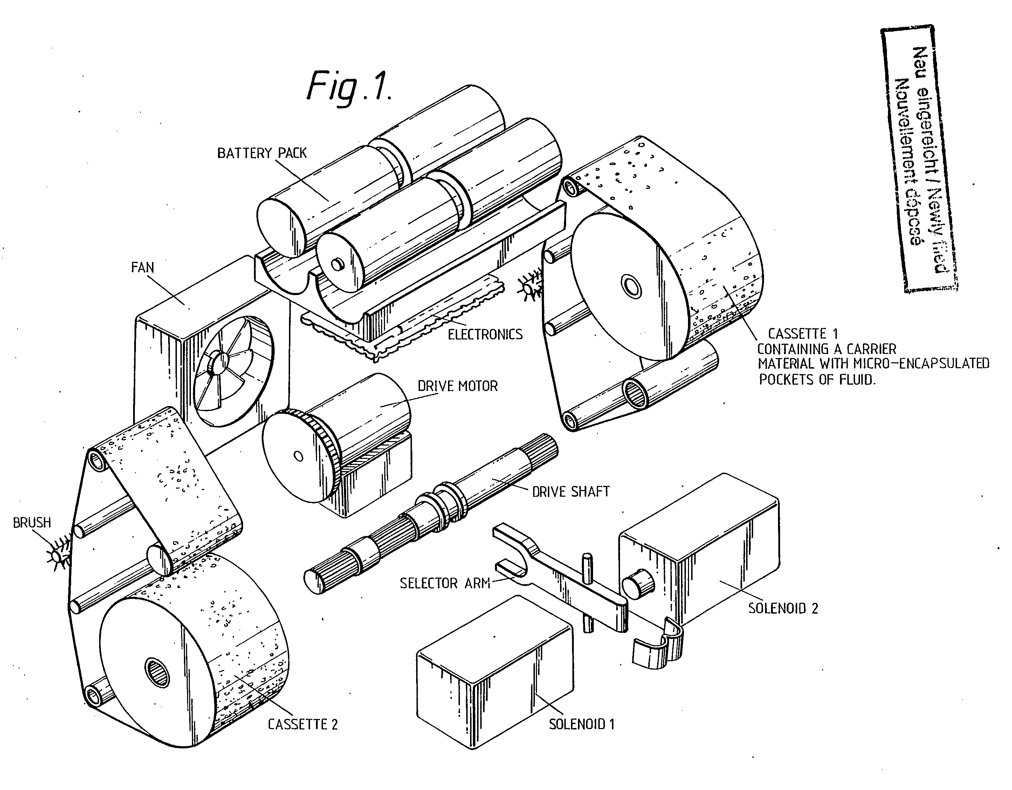

The casing also houses supports for two adjacent webs 12, 14 each extending from an initial roll 12a, 14a to a wind-up roll 12b, 14b. In Figure 1, roll 12a is virtually spent and roll 12b of near maximum size but roll 14a is a fresh roll with little or no material on the wind-up roll 14b. Between the rolls 12b, 14b is a splined drive shaft 16 having an end 16a engaging the core of the roll 12b and an end 16b ready for drivable engagement with the core of roll 14b when a selector arm 18 is moved by a solenoid 20a. Drive to the shaft 16 occurs via a pinion 22 and an electric motor 24. The energisation of the electric motor 24 is controlled by a PC board 26 and batteries 28.

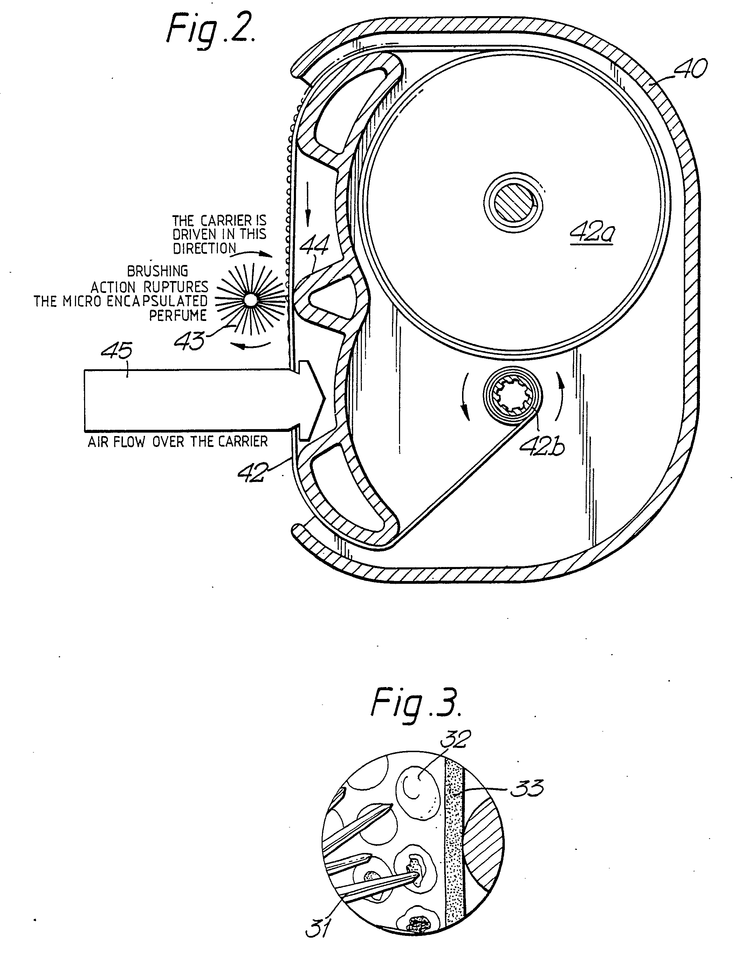

The operation of the illustrated unit involves a slow continuous or periodic advance of one of the webs 12 or 14 from its initial roll 12a, 14a to its wind-up roll 12b, 14b past a dispensing station 12c, 14c. Each dispensing station includes a brush 30 designed to abrade the web and rupture pockets of fluid formed on the outer surface of each web. The pockets have not been shown in Figure 1 since they could range in size from near invisible microcapsules to large blisters occupying a substantial part of the width of the web. Figure 3, however, shows how bristles 31 of the brush 30 rupture microcapsules 32 on a web 33 to release volumes of air-freshening material. With any size of pocket the operation is basically the same, the web 12 or 14 being advanced through the station 12c, 14c and the released material being vaporised in air flows induced by the fan 10 from the brush and/or from the web.

In a typical case, each web 12, 14 could contain sufficient length of material to run for about a month so that two fresh webs could provide about two months (eight weeks) unattended operation. An advance rate of 3 mm of web every 5 minutes would require something close to 50 metres for eight weeks. Thus two 25 metre rolls could be used.

The appropriate one of the solenoids 20a, 20b can be actuated when an initial roll 12a, 14a is exhausted to switch drive over to the other take-up roll 14b, 12b. The solenoids can be controlled on the basis of, inter alia, the diameter of the initial or take-up rolls or tension in the web exceeding a present acceptable running tensions, and are energised via the PC board 26.

Using two webs side-by-side enables a narrower unit to be mounted on the wall without having to reduce the interval between service visits.

The webs can have a uniform distribution of liquid-filled pockets and could be advanced at a uniform rate. However, the rate at which pockets are ruptured can be varied to be different at different times by moving a web with a constant volume of pocketed material per unit length of web at different rates of advance during said different times.

Alternatively the webs 12 and 14 could be provided with different concentrations of fluent material per unit of web area, and one web used for day/weekday and the other for night/weekend use and the selector arm 18 moves as required so that the correct web is used at the correct time.

The web(s) can be advanced a suitable amount on each happening of a specified event as previously discussed.

Although a long wound-up web is currently preferred as the support for the pockets of material to be released it is possible to produce a dispensing unit which uses a plurality of separate web sheets fed in sequence through the dispensing station. Where the web material is strong enough to safely bear the tension required to draw it through the dispensing station, the force to cause transport of the web can be applied downstream, of the dispensing station (as shown in Figure 1). An alternative would be to apply the transport force at the dispensing station or even upstream of the dispensing station if the web is stiff enough.

If necessary, the leading end of a new web could be joined to the trailing end of an almost used-up web by the method described in our copending European Application 88306740.7. In this way webs can follow one another through the dispensing station without interruption.

A long web supporting pockets of fluent air freshening material can be housed in a cassette for use in a dispensing unit and Figure 2 shows a cross-section through such a cassette. In Figure 2 the cassette has been indicated at 40 the web at 42, the initial roll at 42a and the wind-up roll at 42b. The rotating brush which causes rupture of the material-filled pockets is shown at 43 and a web-support surface, against which the brush co-acts, is shown at 44. The arrow 45 indicates air flow past the web 42 downstream of the dispensing station created by the brush 43 and the surface 44.

If the arrow 45 were to indicate a flow of liquid (e.g. water) leading to a pipe or duct the dispensing unit shown could be used to dose the liquid with metred amounts of an additive contained in the pockets.

## Claims

1. A method of dispensing a fluent material, particularly a volatile liquid required for treatment of ambient air, which method comprises conveying a web incorporating a multitude of frangible material-filled pockets past a dispensing station and releasing the fluent material from the pockets by rupturing them at the dispensing station.

2. A method as claimed in claim 1, in which the web includes a substantially uniform volume of fluent material per unit of web length and means is provided to advance the web through the dispensing station at a uniform rate.

3. A method as claimed in claim 1 or claim 2, in which on rupture of each pocket the released material flows onto and is absorbed by the web.

4. A method as claimed in any preceding claim, in which means is provided to move air past the web to entrain vapour from the released material.

5. A method as claimed in any preceding claim, in which a web with a uniform distribution of liquid-filled pockets is advanced at a uniform rate through the dispensing station.

6. A method as claimed in any of claims 1 to 4, in which the rate at which pockets are ruptured is made to be different at different times by moving a web with a constant volume of pocketed material per unit length of web at different rates of advance during said different times.

7. A method as claimed in any of claims 1 to 4, in which the web is provided with different concentrations per unit of web area of equal sized pockets over regions indicated as being suitable for day/night use and/or weekday/weekend use and the correct part of the web passes through the dispensing station at the correct time.

8. A method as claimed in claim 1, in which the web is advanced a suitable amount on each happening of a specified even in a room to match the release of vapour-generating material to the actual use of a room to properly condition the air therein.

9. A method as claimed in claim 1, in which means is provided to pass a fluid flowing in a pipe or duct past the web to entrain the released fluent material.

10. A dispensing unit for dispensing a volatile material into a flow of air, comprising means to advance a web incorporating a multitude of frangible material-filled pockets through a dispensing station, means to rupture the pockets in the dispensing station and means to generate a flow of air past the material released from the ruptured pockets.

11. A unit as claimed in claim 10, which comprises means to support a first web and a second web, the second web acting as a stand-by for use when the first web has been passed through the dispensing station.

12. A unit as claimed in claim 11, in which means is provided to adhere a leading end of the stand-by web to the trailing end of the first web so that both webs pass in sequence through a common dispensing station.

13. A unit as claimed in claim 11, in which two dispensing stations are provided one for each web.

14. A unit as claimed in any one of claims 10 to 13, in which an electric or clockwork motor is provided for advancing the web(s) through the dispensing station(s).

A unit as claimed in any one of claims 10 to 14, in which the dispensing station includes a rotating brush or the like pocket-rupturing member.

16. A unit as claimed in any one of claims 10 to 15, in which a fan is provided to encourage distribution of vapour from the material leaving the ruptured pockets into the flow of air.

17. A web containing pockets of fluent material when used in the method of claim 1 or a dispensing unit according to claim 10.

18. A web as claimed in claim 17, in which the pockets are microcapsules.

19. A web as claimed in claim 17 or 18, in which the web is rolled up on a core.

20. A web as claimed in any one of claims 17 to 19, in which the web is of fibrous material.

Fig.1.

BATTERY PACK

ELECTRONICS

DRIVE MOTOR

DRIVE SHAFT

SELECTOR ARM

SOLENOID 2

SOLENOID 1

CASSETTE 1
CONTAINING A CARRIER
MATERIAL WITH MICRO-ENCAPSULATED
POCKETS OF FLUID.

FAN

CASSETTE 2

BRUSH

## Fig.2.

THE CARRIER IS
DRIVEN IN THIS
DIRECTION

BRUSHING
ACTION RUPTURES
THE MICRO ENCAPSULATED
PERFUME

AIR FLOW OVER THE CARRIER

## Fig.3.